# EUROPÄISCHE PATENTSCHRIFT

(11) **EP 0 772 776 B1**
(45) Veröffentlichungstag und Bekanntmachung des Hinweises auf die Patenterteilung: **22.03.2000**
(21) Anmeldenummer: 95927635.3
(22) Anmeldetag: 27.07.1995
(51) Int. Cl.: G01N 33/543, G01N 33/553, C12Q 1/68, C12N 11/00, A61K 9/50, B03C 1/00

(54) **SUPERPARAMAGNETISCHE TEILCHEN, VERFAHREN ZUR HERSTELLUNG UND DEREN VERWENDUNG**
SUPERPARAMAGNETIC PARTICLES, PROCESS FOR THEIR PRODUCTION AND THEIR USE
PARTICULES SUPERPARAMAGNETIQUES, LEUR PROCEDE DE PRODUCTION ET LEUR UTILISATION

(30) Priorität: 27.07.1994 DE 4427821
(43) Veröffentlichungstag der Anmeldung: 14.05.1997
(73) Patentinhaber: Pilgrimm, Herbert Dr., 14169 Berlin (DE)
(72) Erfinder: Pilgrimm, Herbert Dr., 14169 Berlin (DE)
(74) Vertreter: Walter, Wolf-Jürgen
(86) Internationale Anmeldenummer: DE9501028
(87) Internationale Veröffentlichungsnummer: WO9603653

(56) Entgegenhaltungen:
- EP-A- 0 156 537
- EP-A- 0 176 638
- EP-A- 0 184 710
- EP-A- 0 284 549
- EP-A- 0 321 322
- EP-A- 0 332 022
- WO-A-93/26019
- WO-A-94/09368
- WO-A-94/11103

## Beschreibung

Die Erfindung betrifft superparamagnetische Teilchen, die aus superparamagnetischen Eindomänenteilchen und Aggregaten von superparamagnetischen Eindomänenteilchen aus Eisenoxiden, Eisenmischoxiden oder Eisen bestehen und die auf ihrer Oberfläche organische Substanzen gebunden haben, die gegebenfalls weitere Bindungsstellen zur Kopplung von gewebespezifischen Bindungssubstanzen, diagnostischen oder pharmakologisch wirksamen Substanzen besitzen. Im Patent EP-B-0284549 werden superparamagnetische Eindomänenteilchen aus Eisenoxid-, Eisenmischoxid- oder Eisen mit einer Teilchengröße im Bereich zwischen 3 und 20 Nanometer beschrieben, die auf ihrer Oberfläche organische Substanzen der Gruppe der phosphat-, diphosphat-, polyphosphat-, thiophosphat-, phosphonat- oder thiophosphonatgruppenhaltige Polyalkylenglykole, phosphatgruppenhaltige Nucleotide, deren Oligomere oder deren Polymere sowie phosphatgruppenhaltige Kohlehydrate chemisch gebunden tragen, die weitere Bindungsstellen haben können.

Aus WO 93/26019 sind Magnetteilchen bekannt, die über organo-metallische Polymere gebundene kovalente Gruppen tragen. Die WO 94/09368 beschreibt ferromagnetische Teilchen mit darauf gebundener Bloom-Gelatine und Aminodextran sowie kovalenten Gruppen. Teilchen im Mikrometerbereich aus magnetisierbaren Polymerteilchen mit Liganden mit Bindungsaffinität zu Biopolymeren wie Nukleinsäuren sind in der WO 94/11103 offenbart. Die EP-A-176638 beschreibt eine ferromagnetische Substanz mit einem vernetzten Trägermaterial. In der EP-A-156537 werden superparamagnetische Teilchen offenbart, die kovalente Gruppen über biologische oder biologisch kompatible Polymere wie Albumin, Hefemannan, polymere Saccharose, Dextran oder Polymethylmethacrylat haben und zur Trennung Krebszellen/Normalzellen bei Knochenmarktransplantaten verwendet werden. Die EP-A-184710 betrifft ein koaguliertes Matrixprotein mit inkorporiertem ferromagnetischem Material für bestimmte Trennverfahren. Aus der EP-A-321322 sind magnetisierbare Teilchen mit einem darauf gebundenen Organopolysiloxan bekannt. Die EP-A-332022 beschreibt ein ferromagnetisches Proteinkonjugat, basierend auf Aminogruppen, einer Zwischenstruktur und einem Protein mit SH-Gruppen, das in diagnostischen Verfahren eingesetzt werden kann.

Der Erfindung liegt die Aufgabe zugrunde, den Bereich der Substanzen, die an der Oberfläche der Eindomänenteilchen gebunden sein können, zu erweitern, um die physiko-chemischen und physiologischen Eigenschaften der entstehenden Magnetteilchen den jeweiligen Anwendungsgebieten optimal anpassen zu können, wobei diese Substanzen stabil und leicht herstellbar sein sollen.

Überraschend wurde gefunden, daß mono und polyhydroxylgruppenhaltige aromatische Substanzen, Orthokieselsäure und deren Kondensationsprodukte mit zwei und mehrwertigen anorganischen Ionen und/oder organischen Säuren und Basen und Ortho- oder Metaphosphorsäure und deren Kondensationsprodukte mit der Oberfläche der superparamagnetische Teilchen stabile Bindungen eingehen, die zu stabilen magnetischen Flüssigkeiten und aggregationsstabilen, superparamagnetischen Aggregate führen.

Erfindungsgemäß erfolgt die Stabilisierung der Magnetteilchen durch eine Bindung von mono- und polyhydroxylgruppenhaltigen aromatischen Substanzen, wie z.B. Benzenoiden, Cumarinen, Lignanen, Terphenylen, Flavonoiden, Tanninen, Xanthonen, Benzophenonen, Naphthalenen, Naphthochinonen, Anthrachinonen, Anthracyclinen, polycyclischen kondensierten aromatischen Verbindungen und deren phosphat-, diphosphat-, polyphosphat-, thiophosphat-, phosphonat-, thiophosphonat-, carboxylat-, sulfat-, mercapto-oder silantriolgruppenhaltigen Derivaten, von Silikatgruppenhaltigen Substanzen der Orthokieselsäure und deren Kondensationsprodukten mit zwei und mehrwertigen anorganischen Ionen und/oder organischen Säuren und Basen, von phosphatgruppenhaltigen Substanzen der Ortho- oder Metaphosphorsäure und deren Kondensationsprodukte, auf der Oberfläche der Magnetteilchen.

Neben diesen Stabilisatorsubstanzen können auf der Oberfläche der Magnetteilchen noch zusätzliche organische Substanzen gebunden werden, um die Eigenschaften der Magnetteilchen den gewünschten Anforderungen besser anpassen zu können. Dazu zählen organische Substanzen der Gruppe der phosphat-, diphosphat-, carboxylat-, polyphosphat-, thiophosphat-, phosphonat-, thiophosphonat-, sulfat-, sulfonat-, mercapto-, carboxylat-, silantriolgruppenhaltigen Polyalkylenglykole, und Kohlehydrate, der phosphatgruppenhaltigen Nucleotide, deren Oligomere oder deren Polymere, der Alkyl-, Aryl-, und/oder Alkyl-aryl-polyethylenglykol- phosphate oder -phosphonate, der Gruppe der stickstoffhaltigen Polysaccharide, ausgewählt unter Mucopolysacchariden, Glykoproteiden, Chitinen, sowie deren Derivaten und Denaturierungsprodukten.

Beispielhafte Stabilisatorsubstanzen für mono- und/oder polyhydroxylgruppenhaltige aromatische Substanzen sind Caffeesäure, Gallussäure, Hexahydroxydiphensäure, Ellagsäure, Chebulsäure, und deren Derivate und Kondensationsprodukte mit Kohlenhydraten und Phenolkarbonsäuren, Aesculin, Rutin, Aescin Troxerutin, Hesperidin, Aloin, Kaempferol, Quercetin, Gallotannine, Ellagitannine, Ruberythrinsäure, Carminsäure, natürliche und synthetische Farbstoffe wie Anthrachinon- oder Phthalocyanihfarbstoffe, Daunorubicin, Ansamycin sowie deren phosphat-, diphosphat-, polyphosphat-, thiophosphat-, phosphonat-, thiophosphonat-, carboxylat-, sulfat-, mercapto- oder silantriolgruppenhaltigen Derivate.

Beispielhafte Stabilisatorsubstanzen für silikatgruppenhaltige Kondensationsprodukte der Orthokieselsäure mit zwei und mehrwertigen anorganischen Ionen sind die Kondensationsprodukte mit den Elementen Al, Au, Bi, Cr, J, Mo, P, Pt, Se, Tc, Ti, Y, Zr und seltene Erdmetalle.
Beispielhafte Stabilisatorsubstanzen für silikatgruppenhaltige Kondensationsprodukte der Orthokieselsäure mit organischen Säuren und Basen sind deren Kondensationsprodukte mit Phytinsäure, Alginsäure, Gallussäure.
Beispielhafte Stabilisatorsubstanzen für phosphatgruppenhaltige Kondensationsprodukte der Ortho- oder Metaphosphorsäure sind Pyrophosphorsäure, Polyphosphorsäuren, Cyclophosphate und Heterokondensationsprodukte und wasserunlösliche Salzverbindungen mit anorganischen Ionen, wie Ag, Au, Bi, Mo, Pt, Tc, Y, Zr, und basische Gruppen enthaltende organische Verbindungen, wie Spermin, Spermidin, Polyethylenimin, Oxygelatine.

Die Stabilisatorsubstanzen sind nach dem Stand der Technik herstellbar oder können käuflich erworben werden.

Erfindungsgemäß können an die Stabilisatormoleküle, die mit ihren hydroxylgruppenhaltigen aromatischen Substanzen, silikatgruppenhaltigen Substanzen und phosphatgruppenhaltige Substanzen an der superparamagnetischen Teilchenoberfläche gebunden sind, gewebespezifische Bindungssubstanzen, wie z.B. Antigene, Antikörper, Ribonucleinsäuren, Desoxyribonucleinsäuren, Ribonucleinsäuresequenzen, Desoxyribonucleinsäuresequenzen, Haptene, Avidin, Streptavidin, Protein A, Protein G, Endotoxin-bindende Proteine, Lectine, Selectine, pharmakologisch wirksame Substanzen, wie z.B. Antitumorproteine, Enzyme, Antitumorenzyme, Antibiotika, Pflanzenalkaloide, Alkylierungsreagenzien, Antimetaboliten, Hormone und Hormonantagonisten, Interleukine, Interferone, Wachstumsfaktoren, Tumornekrosefaktoren, Endotoxine, Lymphotoxine, Urokinase, Streptokinase, Plasminogen- Streptokinase-Aktivator-Komplex, Gewebe-Plasminogen-Aktivatoren, Desmodus-Plasminogen-Aktivatoren, Makrophagen-Aktivierungs-körper, Antisera, Proteaseninhibitoren, radioaktive Isotope enthaltende Substanzen, Tenside, kardiovaskulare Pharmazeutika, Chemotherapeutika, gastrointestinale Pharmazeutika, Neuropharmazeutika, pharmakologisch wirksame Zellen, wie z.B. Organellen, Viren, Mikroben, Algen, Pilze, insbesondere Erythrozyten, Thrombozyten, Granulozyten, Monozyten, Lymphozyten, Langerhans'sche Inseln, pharmakologisch wirksame Komplexbildner, wie z.B. Polycarbonsäuren, Polyaminocarboxylsäuren, Porphyrine, Katecholamine, zellfusionvermittelnde Substanzen, wie z.B. Polyethylenglykole, Alkyl-polyethylenglykole, Alkyl-arylpolyethylenglykole, mit Wasser mischbare Polypropylenglykole (PPG)ₘ oder mit Wasser mischbare Block-Copolymerisate aus Polyethylenglykol (PEG) und Polypropylenglykol (PPG), ausgewählt unter den Block-Copolymerisaten (PEG)ₙ-(PEG)ₘ, (PEG)ₙ-(PPG)ₘ-(PEG)ₙ, (PPG)ₘ-(PEG)ₙ-(PPG)ₘ, wobei n und m positive ganze Zahlen sind, ausgewählt für PEG im Bereich von 4 bis 1000, für PPG im Bereich von 3 bis 12 und für PEG-PPG-Block-Copolymerisate im Bereich von 3 bis 140, und/oder gentransfer-vermittelnden Substanzen, wie z.B. Polyethylenglykole und deren Derivate und Polyalkylenimine, wie z.B. Pentaethylenhexamin, Polyethylenimin, Spermin, Spermidin gebunden werden.

Die erfindungsgemäßen, mit hydroxylgruppenhaltigen aromatischen Stabilisatorsubstanzen stabilisierten superparamagnetischen Teilchen adsorbieren relativ fest aminosäurehaltige und aromatische Verbindungen, so daß für einige Anwendungsfälle eine reine adsorptive Bindung von gewebespezifische Bindungssubstanzen, pharmakologisch wirksame Substanzen und pharmakologisch wirksame Zellen ausreichend ist, um sie für ein magnetischen drug targeting oder als Kontrastmittel einsetzen zu können.

Die erfindungsgemäßen, mit silikatgruppenhaltigen Substanzen der Orthokieselsäure und deren Kondensationsprodukten mit organischen Säuren und Basen, stabilisierten superparamagnetischen Teilchen sind für adsorptive Bindungen und für viele Kopplungsreaktionen verwendbar, bei denen die Reaktivität der funktionellen Gruppen der organischen Säuren und Basen, die mit den silikatgruppen stabile Kondensationsprodukte gebildet haben, für eine chemische Bindung von gewebespezifische Bindungssubstanzen, pharmakologisch wirksame Substanzen, pharmakologisch wirksame Zellen, pharmakologisch wirksamen Komplexbildner, zellfusionvermittelnden Substanz und/oder gentransfer-vermittelnden Substanz anwendbar ist. Die Kopplung gewebespezifischer Bindungssubstanzen, pharmakologisch wirksamer Substanzen, pharmakologisch wirksamer Zellen, pharmakologisch wirksamer Komplexbildner, zellfusionvermittelnder Substanzen oder gentransfer-vermittelnder Substanzen an die superparamagnetischen Teilchen hat weiterhin den Vorteil, daß Über die Relaxationszeitveränderungen der resonanzfähigen Wasserstoffatome im Körper der Therapiefortschritt mit der Kernspin-Diagnostik beobachtet werden kann.

Die Herstellung der superparamagnetischen Teilchen erfolgt durch eine Fällung aus einer Eisensalzlösung mit Alkalilauge oder Ammoniakwasser und einer nachfolgenden gezielten Agglomeration der entstandenen superparamagnetischen Eindomänenteilchen. Dabei werden die superparamagnetischen Eindomänenteilchen in Wasser verrührt und bei einem pH-Wert von 1 bis 7 durch Erhitzen auf 80 bis 120°C, bei Temperaturen über 100°C im Autoklaven, zur Aggregation gebracht.
Nach dem Abkühlen der Dispersion werden die Teilchen so lange gewaschen, bis die elektrische Leitfähigkeit des Filtrates < 10 µS/cm beträgt. Die so hergestellten superparamagnetischen Teilchen bilden sofort einen schnell sedimentierenden Niederschlag, der sich auch durch starkes Rühren oder durch Ultraschallbehandlung nicht in eine stabile Dispersion überführen läßt. Erst die Bindung von Stabilisatorsubstanzen auf der Oberfläche der superparamagnetischen Teilchen sorgt für eine Dispergierbarkeit.
Bei einigen Stabilisatorsubstanzen reicht Rühren mit dem Glasstab, bei anderen Stabilisatorsubstanzen benötigt man einen stärkeren Energieeintrag, wie z.B. Erwärmen oder Einwirkung von Ultraschall, um stabile Dispersionen zu erhalten.
Je nach Anwendungsgebiet können die magnetischen Dispersionen dialysiert werden, um den überschüssigen Anteil an Stabilisatorsubstanz zu entfernen.
Die stabilisierten superparamagnetischen Teilchendispersionen enthalten die noch nicht oder nur schwach aggregierten superparamagnetischen Eindomänenteilchen. Diese bilden eine stabile magnetische Flüssigkeit, die sich leicht von den größeren superparamagnetischen Teilchen durch deren Sedimentation in einem Magnetfeld entsprechender Stärke und Inhomogenität abtrennen lassen. Die stabilisierten superparamagnetischen Eindomänenteilchen lassen sich gut als Kontrastmittel für die Kernspin-Diagnostik, als zellfusionvermittelnde Substanzen oder als gentransfer-vermittelnde Substanzen verwenden, wobei auch hier die Wirksamkeit der Zellfusion und des Gentransfers mit der Kernspin-Diagnostik untersucht werden kann.

In einer einfachen Ausführung der magnetischen Separation stellt man ein Becherglas mit der magnetischen Dispersion auf einen Permanentmagneten mit einer magnetischen Flußdichte von 0,1 T und gießt nach einer Sedimentationszeit von ca.30 min die überstehende magnetische Flüssigkeit ab. Zurück bleiben die superparamagnetischen Aggregate, die, je nach Teilchengröße, sich wieder spontan in der Dispersion verteilen oder als Bodensatz im Becherglas zurückbleiben. Bis zu Teilchengrößen von ungefähr 500 nm verteilen sich die superparamagnetischen Teilchen wieder spontan oder unter leichtem Rühren im wäßrigen Dispersionsmittel.
Die Sedimentationsstabilität der erfindungsgemäßen superparamagnetischen Aggregate ist wesentlich höher als bei den bisher bekannten Magnetteilchen mit vergleichbaren magnetischen Eigenschaften, was wahrscheinlich auf die starke Strukturierung der die superparamagnetischen Teilchen umgebenden Wassermoleküle und den damit vergrößerten Stokes'schen Teilchendurchmesser zurückzuführen ist.

Da der Anteil an superparamagnetischen Eindomänenteilchen in den superparamagnetischen Aggregaten wesentlich höher als bei den bisher bekannten Magnetteilchen ist, ist auch die Abscheidungsgeschwindigkeit der superparamagnetischen Aggregate in einem inhomogenen Magnetfeld größer. In einer 10 Gew.-% igen wäßrigen Dispersion von superparamagnetischen Teilchen, mit einem Durchmesser von ca 100 nm und einem Magnetitanteil von 95 %, beträgt die Abscheidungszeit der Magnetteilchen auf einen Permanentmagneten mit einer magnetischen Flußdichte von 0,1 T weniger als 1 min.

Die erfindungsgemäßen superparamagnetischen Teilchen als Aggregate haben Eisenoxidgehalte von 90 bis 98 Gew.-%. Gegenüber dem Stand der Technik, daß Magnetteilchen bis zu 60 Gew.-% Eisenoxid enthalten können, bedeutet das eine wesentliche Verbesserung der magnetischen Eigenschaften. Damit können die neuen superparamagnetischen Teilchen, bei gleicher magnetischen Wechselwirkung, entsprechend kleiner als die bisher bekannten Magnetteilchen sein. Die spezifische Oberfläche vergrößert sich, es können mehr pharmakologisch wirksame Substanzen oder gewebespezifische Bindungssubstanzen auf der Oberfläche gekoppelt werden. Mit Verkleinerung der Teilchengröße wird auch die biologische Verträglichkeit besser, die Abbaugeschwindigkeit im Körper erhöht. Auch die freie verfügbare Zeit der Magnetteilchen beim magnetischen drug targeting, d.h. die Zeit bis die Teilchen vom retikuloendothelialem System gebunden sind, erhöht sich mit Verringerung der Teilchengröße.
Die Bioverfügbarkeit der superparamagnetischen Teilchen im Körper beträgt, je nach Teilchengröße und Zusammensetzung der Stabilisatorsubstanzen nur wenige Minuten bis einige Stunden d.h. das retikuloendotheliale System bindet die superparamagnetischen Teilchen relativ schnell.

An Beispielen sollen die Herstellung der erfindungsgemäßen superparamagnetischen Teilchen erläutert werden

### Beispiel 1:

Eisen (III)-chlorid (270 g) und Eisen(II)-sulfat (153 g) werden in 1 l dest. Wasser gelöst. Durch Zugabe von Natronlauge wird unter Rühren ein pH-Wert von 9,5 eingestellt. Nach erfolgter Fällung wird die Dispersion unter Rühren mit Salzsäure auf den pH-Wert von 5,0 eingestellt und auf 100°C erwärmt. Nach dem Abkühlen der Dispersion wird der Niederschlag gewaschen, bis das Filtrat eine elektrische Leitfähigkeit von < 10 µS/cm besitzt. Die Stabilisierung der superparamagnetischen Teilchen erfolgt durch Mischen einer wäßrigen oder niedrigsiedende polare Lösungsmittel enthaltenden Stabilisatorlösung mit den Magnetteilchen bei Raumtemperatur. Die Stabilisatorlösung kann dabei, je nach den gewünschten Eigenschaften, aus reinen Stabilisatorsubstanzen oder aus Mischungen von Stabilisatorsubstanzen bestehen. Zur Beschleunigung der Dispergierung und Stabilisierung kann die Dispersion gerührt oder mit Ultraschall behandelt werden. Kommen niedrigsiedende organische Lösungsmittel zur Anwendung, werden diese zur Entfernung nach der Stabilisierung durch Vakuumverdampfung oder Dialyse entfernt.

### Beispiel 2:

Eisen(III)-chlorid (270 g) und Eisen(II)-chlorid(119 g) werden in 1 l dest. Wasser gelöst. Durch Zugabe von Ammoniakwasser wird unter Rühren der pH-Wert der Lösung auf 9,6 eingestellt. Nach erfolgter Fällung wird die Dispersion mit Salzsäure auf den pH 6,0 eingestellt, mit 20 ml 30%-igem Wasserstoffperoxid versetzt und auf 100°C erwärmt. Nach dem Abkühlen wird der Niederschlag mit dest. Wasser gewaschen, bis das Filtrat eine elektrische Leitfähigkeit von < 10 µS/cm besitzt. Das entstehende γ-Fe₂O₃ kann stabilisiert werden.

### Beispiel 3:

Eisen (III)-chlorid (270 g) und Eisen(II)-sulfat (153 g) werden in 1 l dest. Wasser gelöst. Durch Zugabe von Natronlauge wird unter Rühren ein pH-Wert von 9,5 eingestellt. Nach erfolgter Fällung wird die Dispersion unter Rühren mit 20g Polyphosphorsäure versetzt und auf 100°C erwärmt. Nach dem Abkühlen der Dispersion wird der Niederschlag gewaschen, bis das Filtrat eine elektrische Leitfähigkeit von < 10 µS/cm besitzt. Der Feststoff wird in 300 ml Wasser verrührt und 20 min mit Ultraschall von 100 W Leistung dispergiert. Die entstehende Dispersion wird 30 min auf einen Permanentmagneten mit einer magnetischen Flußdichte von 0,1 T sedimentiert und der Überstand von magnetischer Flüssigkeit abgegossen. Der Überstand enthält überwiegend stabilisierte superparamagnetische Eindomänenteilchen. Das Sediment auf dem Permanentmagneten enthält die superparamagnetischen abbaubaren Aggregate. Durch mehrmaliges Waschen mit dest. Wasser und erneuerter Sedimentation im Magnetfeld können die superparamagnetischen Aggregate rein und in enger Teilchengrößenverteilung erhalten werden. Die superparamagnetischen Teilchen haben einen mittleren Teilchendurchmesser von ca.80 nm.

### Beispiel 4:

Die gesamte Menge des mit Polyphosphorsäure stabilisierten und gewaschenen Magnetit-Niederschlages von Beispiel 3 wird unter Rühren in 300 ml Wasser verrührt, mit 10 %-iger Salzsäure auf pH 1,0 eingestellt, eine Mischung von 28,4 g Bismuthchlorid in 20 ml konz. Salzsäure dazugegeben und anschließend mit Natronlauge der pH von 2,5 eingestellt.In diese Dispersion wird eine Lösung von 30 g Natriumsilikat in 200 ml dest. Wasser verrührt und 10 min mit Ultraschall von 100 W Leistung dispergiert. Die Abtrennung der nicht oder nur schwach agglomerierten superparamagnetischen Eindomänenteilchen, die eine stabile magnetische Flüssigkeit bilden, erfolgt durch eine magnetische Sedimentation wie im Beispiel 3 beschrieben. Die superparamagnetischen Eindomänenteilchen und die superparamagnetischen Aggregate sind sehr gut als orales Kontrastmittel für die Kernspin- und Röntgen-Diagnostik anwendbar.

### Beispiel 5:

Die gesamte Menge des Magnetit-Niederschlages von Beispiel 1 wird zur Stabilisierung in eine Lösung von 30 g Gallussäure in 400 ml dest. Wassern verrührt und 10 min mit Ultraschall von 100 W Leistung dispergiert. Die Abtrennung der nicht oder nur schwach agglomerierten superparamagnetischen Eindomänenteilchen, die eine stabile magnetische Flüssigkeit bilden, erfolgt durch eine magnetische Sedimentation wie im Beispiel 3 beschrieben. Die superparamagnetischen Aggregate sind sehr gut für die magnetische Anreicherung in Tumoren geeignet. Hier können sie durch magnetomechanische Immunstimulierung, oder zusätzlich durch Hyperthermie, d.h. durch Einstrahlung elektromagnetischer Strahlung und Erwärmung des Tumors, den Tumor zerstören. Die superparamagnetischen Eindomänenteilchen sind als orales oder i.v. Kontrastmittel für die Kernspin-Diagnostik anwendbar.

### Beispiel 6:

Die gesamte Menge des Magnetit-Niederschlages von Beispiel 1 wird in eine Lösung von 40g Methoxy-polyethylenglykol-phosphat (MG 2000) und 20 g Rutinosid in 300 ml Methanol gegeben und 5 min mit Ultraschall von 100 W Leistung dispergiert. Anschließend wird zur Dispersion 500 ml dest. Wasser gegeben und das Methanol abdestilliert. Die wäßrige Dispersion wird nochmals 20 min mit Ultraschall von 100 W Leistung dispergiert. Die Abtrennung der nicht oder nur schwach agglomerierten superparamagnetischen Eindomänenteilchen, die eine stabile magnetische Flüssigkeit bilden, erfolgt durch eine magnetische Sedimentation wie im Beispiel 3 beschrieben. Die superparamagnetischen Aggregate sind für eine parenterale Tumorschädigung einsetzbar, da sich, wie in Versuchen mit Nacktmäusen und Ratten gezeigt hat, eine starke Aktivierung des Immunsystems einsetzt, die zur totalen Remission von Tumoren führen kann. Des weiteren sind die superparamagnetischen Aggregate für eine Kopplung von aminosäurehaltigen gewebespezifischen Bindungssubstanzen, pharmakologisch wirksamen Substanzen und pharmakologisch wirksamen Zellen geeignet. Die superparamagnetischen Eindomänenteilchen sind als parenterales Kontrastmittel für die Kernspin-Diagnostik zur Darstellung des Gefäßsystemes und des Magen-Darm-Traktes oder als Vektor für den Gentransfer anwendbar.

### Beispiel 7:

20 ml der Dispersion der superparamagnetischen Aggregate von Beispiel 6, mit einer magnetischen Sättigungsinduktion von 10 mT, werden mit einer Lösung von 10 mg Doxorubicin in 10 ml physiologische Kochsalzlösung gemischt. Diese superparamagnetischen Teilchen sind sehr gut für die magnetische Anreicherung in Tumoren geeignet. Die starke Aktivierung des Immunsystemes durch die superparamagnetischen Teilchen kann zu einer Tumorschädigung führen, die durch die Wirkung des Zytostatikum verstärkt werden kann.

### Beispiel 8:

Die gesamte Menge des γ-Fe203-Niederschlages von Beispiel 2 wird in eine Lösung von 30 g Tanninsäure in 500 ml Wasser gegeben und 5 min gerührt und 10 min mit einem Ultraschalldispergator (100 W Leistung) dispergiert. Die entstehende Dispersion wird mit einem 50 kD-Filter gegen dest. Wasser dialysiert, um überschüssige Stabilisatorsubstanzen zu entfernen. Die Abtrennung der nicht oder nur schwach agglomerierten superparamagnetischen Eindomänenteilchen, die eine stabile magnetische Flüssigkeit bilden, erfolgt durch eine magnetische Sedimentation wie im Beispiel 3 beschrieben. Die entstehenden superparamagnetischen Aggregate haben einen mittleren Teilchendurchmesser von ca. 80 nm und sind für die Kopplung von aminosäurehaltigen gewebespezifischen Bindungssubstanzen, pharmakologisch wirksamen Substanzen und pharmakologisch wirksamen Zellen geeignet. Die superparamagnetischen Eindomänenteilchen sind als orales oder i.v. Kontrastmittel für die Kernspin- Diagnostik anwendbar.

### Beispiel 9:

20 ml der superparamagnetischen Aggregate von Beispiel 8, mit einer magnetischen Sättigungsinduktion von 10 mT, werden mit einer Lösung von 10 mg Mitoxantron in 20 ml einer acetatgepufferten physiologische Kochsalzlösung gemischt.
Diese superparamagnetischen Teilchen sind sehr gut für die magnetische Anreicherung in Tumoren geeignet. Hier können sie durch die magnetische Anreicherung im Tumor und die verstärkte Desorption des Zytostatikums unter der Wirkung des inhomogenen Magnetfeldes zur Tumorschädigung führen.

### Beispiel 10:

Die gesamte Menge des Magnetit-Niederschlages von Beispiel 2 wird in eine Lösung von 40 ml einer 40 %-igen Phytinsäurelösung in 500 ml dest. Wasser gegeben, 5 min gerührt und 10 min mit einem Ultraschalldispergator (100 W Leistung) dispergiert. Die entstehende Dispersion wird mit einer 30 %-igen Natriumsilikatlösung auf einen pH-Wert von 7,0 titriert, 10 min mit einem Ultraschalldispergator (100 W Leistung) dispergiert und 30 min auf einen Permanentmagneten mit einer magnetischen Flußdichte von 0,1 T sedimentiert und der Überstand von magnetischer Flüssigkeit abgesaugt. Das Sediment auf dem Magnetfeld enthält die superparamagnetischen Teilchen. Durch mehrmaliges Waschen mit dest. Wasser und erneuert Sedimentation im Magnetfeld können die superparamagnetischen Teilchen rein und in enger Teilchengrößenverteilung erhalten werden. Die superparamagnetischen Teilchen haben einen mittleren Teilchendurchmesser von 160 nm und sind für die Kopplung von aminosäurehaltigen gewebespezifischen Bindungssubstanzen, pharmakologisch wirksamen Substanzen und pharmakologisch wirksamen Zellen geeignet.
Die Hauptanwendungsgebiete der erfindungsgemäßen superparamagnetischen Teilchen liegen auf den Gebieten des magnetischen drug targeting, der Kontrastmittel, der Zellfusion und des Gentransfers. Aufgrund der sehr hohen Anteiles an Magnetmaterial ( 90 bis 98 Gew.-%) lassen sich schon kleine Magnetteilchen sehr gut und sehr schnell in bestimmte Regionen des Körpers mit Hilfe von elektromagnetischen oder Permanentmagnet-Feldern konzentrieren. Die erfindungsgemäßen superparamagnetischen Aggregate sind für eine parenterale Tumorschädigung einsetzbar, da bei ihrer Injektion in den Flutkreislauf eine starke Aktivierung des Immunsystemes einsetzt, die zur totalen Remission von Tumoren führen kann. Bei Kopplung von pharmakologisch wirksamen Substanzen an die superparamagnetischen Aggregate, kann deren Konzentration am Wirkungsort erhöht werden, insbesondere beim Einsatz tumorspezifischer Antikörper. Dieser Umstand hat für die Krebstherapie Bedeutung, da die zur Chemotherapie von Tumoren eingesetzten Substanzen sehr starke Nebenwirkungen auf den gesamten Organismus ausüben und bei einer Anreicherung am Wirkungsort der übrige Körper weniger stark mit Zytostatika oder radioaktiven Isotopen belastet wird.
Bei Kopplung von Nucleiden, Nucleinsäuren, Antimetaboliten oder antitumoralen oder antiviralen Derivaten an die superparamagnetischen Aggregate, kann deren Konzentration am Wirkungsort drastisch erhöht werden, insbesondere bei ihrem Einsatz in der Gentherapie. Die superparamagnetischen Teilchen können durch Kopplung an Viren, Bakterien, Zellen und deren Oberflächenmolekülen zur Immunaktivierung im Körper eingesetzt werden, wobei die Einwirkung von Magnetfeldern die Immunaktivierung unterstützt.
Die Menge der einzusetzenden superparamagnetischen Teilchen ist beim magnetischen drug targeting abhängig von der Teilchengröße, von der Zusammensetzung der Stabilisatorsubstanzen, von der Anwesenheit bindungsspezifischer Antikörper und von der magnetischen Feldstärke am Wirkungsort. Die erfindungsgemäßen superparamagnetischen Teilchen können als orale und parenterale Kontrastmittel für die Kernspin-Diagnostik eingesetzt werden. Die Mengen an superparamagnetischen Teilchen liegen bei der Anwendung als parenterales Kontrastmittel für die MRI bei ca.20 µM Fe/kg Körpergewicht und bei der Anwendung als orales Kontrastmittel für die MRI bei ca.10 µM Fe/kg Körpergewicht.
In Tierversuchen konnten gute Kontrasteffekte als parenterales Kontrastmittel für Leber, Milz, Knochenmark und Blutkreislauf, für die Lymphographie, als antikörperspezifische Kontrastmittel für die Tumor- und Thrombendiagnostik und als orales Kontrastmittel für die Abbildung des Magen-Darm-Traktes gefunden werden.
Die superparamagnetischen Teilchen können auch zur in vitro Diagnostik, Zellfusion und zum Gentransfer gegebenenfalls unter Einwirkung von Magnetfeldern, verwendet werden, wenn auf der Oberfläche der Teilchen die entsprechenden diagnostischen, zellfusionvermittelnden und gentransfer-vermittelnde Substanzen gebunden werden. Aufgrund der starken magnetischen Wechselwirkung der superparamagnetischen Teilchen mit Magnetfeldern, lassen sich noch sehr kleine superparamagnetische Teilchen nach erfolgter diagnostischer Reaktion, Zellfusion oder erfolgtem Gentransfer leicht aus dem Reaktionsgemisch wieder abtrennen.
Die superparamagnetischen Teilchen können auch als magnetische Ionenaustauscher und magnetische Adsobentien zur Abtrennung von Ionen, organischen Molekülen, Makromolekülen, Zellen, Viren u.s.w. in der Biotechnologie, Abwasserreinigung oder sonstigen Stofftrennungsverfahren verwendet werden, wenn auf der Oberfläche der Teilchen die entsprechenden Ionenaustauschergruppen und Adsorbentien gebunden werden.

## Patentansprüche

1. Superparamagnetische Teilchen, dadurch gekennzeichnet, daß sie Teilchen umfassen, die ausgewählt sind aus der Gruppe, die aus
(a) kleinen superparamagnetischen Eindomänenteilchen aus Eisenhydroxid, Eisenoxidhydrat, Eisenoxid-, Eisenmischoxid- oder Eisen mit einer Teilchengröße im Bereich zwischen 3 und 50 Nanometer,
(b) stabilen, abbaubaren Aggregaten mit einer Teilchengröße im Bereich zwischen 10 und 1000 Nanometer, wobei die Aggregate aus mehreren kleinen superparamagnetischen Eindomänenteilchen aus Eisenhydroxid, Eisenoxidhydrat, Eisenoxid-, Eisenmischoxid- oder Eisen mit einer Teilchengröße im Bereich zwischen 3 und 50 Nanometer
und deren Gemischen besteht, wobei
(c) die Teilchen auf ihrer Oberfläche Substanzen gebunden tragen, die ausgewählt sind aus der Gruppe, die aus
(i) mono- und/oder polyhydroxylgruppenhaltigen aromatischen Substanzen, ausgewählt unter Benzenoiden, Cumarinen, Lignanen, Terphenylen, Flavonoiden, Tanninen, Xanthonen, Benzophenonen, Naphthalenen, Naphthochinonen, Anthrachinonen, Anthracyclinen, polycyclischen kondensierten aromatischen Verbindungen und deren phosphat-, diphosphat-, polyphosphat-, thiophosphat-, phosphonat-, thiophosphonat-, carboxylat-, sulfat-, mercapto- oder silantriolgruppenhaltigen Derivaten;
(ii) silikatgruppenhaltigen Substanzen der Orthokieselsäure und deren Kondensationsprodukten mit zwei und mehrwertigen anorganischen Ionen oder mit organischen Säuren oder Basen oder Gemischen davon;
(iii) phosphatgruppenhaltigen Substanzen der Ortho- oder Metaphosphorsäure und deren Kondensationsprodukte sowie deren Heterokondensationsprodukte und wasserunlösliche Salzverbindungen mit anorganischen Ionen und basische Gruppen enthaltende organische Verbindungen;
und deren Gemischen besteht;
und wobei die Teilchen auf ihrer Oberfläche gegebenenfalls zusätzlich zu den Substanzen (c) noch organische Substanzen (d) chemisch gebunden tragen, die ausgewählt sind aus der Gruppe, die aus
(iv) phosphat-, diphosphat-, carboxylat-, polyphosphat-, thiophosphat-, phosphonat-, thiophosphonat-, sulfat-, sulfonat-, mercapto-, carboxylat-, silantriol-, trialkoxysilangruppenhaltigen Polyalkylenglykolen und Kohlehydraten;
(v) phosphatgruppenhaltigen Nucleotiden, deren Oligomeren oder deren Polymeren;
(vi) Alkyl-, Aryl-, Alkyl-aryl-polyalkylenglykolphosphaten oder -phosphonaten, Phosphaten oder Phosphonaten der Block-Copolymerisate aus Polyethylenglykol (PEG) und Polypropylenglykol (PPG), ausgewählt unter den Block-Copolymerisaten (PEG)ₙ-(PPG)ₘ, (PEG)ₙ-(PPG)ₘ-(PEG)ₙ, (PPG)ₘ-(PEG)ₙ-(PPG)ₘ;
(vii) stickstoffhaltigen Polysacchariden, ausgewählt unter Mucopolysacchariden, Glykoproteiden, Chitinen, sowie deren Derivaten und Denaturierungsprodukten;
und deren Gemischen besteht.

2. Superparamagnetische Teilchen nach Anspruch 1, dadurch gekennzeichnet, daß die superparamagnetischen Eindomänenteilchen (a) und die Teilchen der stabilen, abbaubaren Aggregate (b) aus Eisenhydroxid, Eisenoxidhydrat, γ-Fe₂O₃, Fe₃O₄, aus den Eisenmischoxiden der allgemeinen Formel mMo.nFe₂O₃, worin M die zweiwertigen Metallionen Fe, Co, Ni, Mn, Be, Mg, Ca, Ba, Sr, Cu, Zn, Pt oder Gemische davon bedeuten, aus den Mischoxiden der allgemeinen Formel mFe₂O₃.nMe₂O₃, worin Me die dreiwertigen Metallionen Al, Cr, Bi, seltene Erdmetalle oder Gemische davon bedeuten, oder Eisen bestehen.

3. Superparamagnetische Teilchen nach einem der Ansprüche 1 bis 2, dadurch gekennzeichnet, daß die Substanzen c) ausgewählt sind unter mono- und/oder polyhydroxylgruppenhaltigen aromatischen Benzenoiden, Cumarinen, Lignanen, Terphenylen, Flavonoiden, Tanninen, Xanthonen, Benzophenonen, Naphthalenen, Naphthochinonen, Anthrachinonen, Anthracyclinen, polycyclischen kondensierten aromatischen Verbindungen und deren phosphat-, diphosphat-, polyphosphat-, thiophosphat-, phosphonat-, thiophosphonat-, carboxylat-, sulfat-, mercapto- oder silantriolgruppenhaltigen Derivaten.

4. Superparamagnetische Teilchen nach einem der Ansprüche 1 bis 3, dadurch gekennzeichnet, daß die Substanzen c) ausgewählt sind unter den silikatgruppenhaltigen Substanzen der Orthokieselsäure und deren Kondensationsprodukten mit zwei und mehrwertigen anorganischen Ionen, ausgewählt unter Al, Au, Bi, Cr, J, Mo, P, Pt, Se, Tc, Ti, Y, Zr, seltene Erdmetalle und/oder organischen Säuren und Basen.

5. Superparamagnetische Teilchen nach einem der Ansprüche 1 bis 4, dadurch gekennzeichnet, daß die Substanzen (c) ausgewählt sind unter den phosphatgruppenhaltigen Substanzen der Ortho- oder Metaphosphorsäure und deren Kondensationsprodukte, sowie deren Heterokondensationsprodukte und wasserunlösliche Salzverbindungen mit anorganischen Ionen, organischen Basen und basische Gruppen enthaltende organische Verbindungen.

6. Superparamagnetische Teilchen nach Anspruch 1, dadurch gekennzeichnet, daß auf ihrer Oberfläche zusätzlich zu den Substanzen (c) noch organische Substanzen (d) gebunden sind, ausgewählt unter (i) den Verbindungen der allgemeinen Formel
X - R - A - B
worin
X eine funktionelle Gruppe darstellt, ausgewählt aus der Alkoxy-, Monolkylamino-, Dialkylamino-, Trialkylamino- und Alkylthiogruppe, bei denen die Zahl der Kohlenstoffatome im Alkylteil dieser Gruppen im Bereich von 1 und 4 liegt, oder eine funktionelle Gruppe darstellt, ausgewählt unter der Hydroxyl-, Amin-, Aldehyd-, Dimethylacetal-, Diethylacetal-, Epoxy-, Thiol-, Carboxy-, 4,6-Dichlortriazin-, Hydroxamsäure-, Isocyanat-, Acylazid-, Anhydrid-, Diazoniumsalz-, Iminocarbonat- und Toluolsulfonatgruppe;
R ist ein Polyethylenglykolrest (PEG)ₙ, ein mit Wasser mischbarer Polypropylenglykolrest (PPG)ₘ oder ein mit Wasser mischbarer Block-Copolymerisatrest aus Polyethylenglykol (PEG) und Polypropylenglykol (PPG), ausgewählt unter den Block-Copolymerisaten (PEG)ₙ-(PPG)ₘ, (PEG)ₙ-(PPG)ₘ-(PEG)ₙ, (PPG)ₘ-(PEG)ₙ-(PPG)ₘ,
n und m sind positive ganze Zahlen, ausgewählt für PEG im Bereich von 4 bis 1000, für PPG im Bereich von 3 bis 12 und für PEG-PPG--Block-Copolymerisate im Bereich von 3 bis 140,
R ist ein Polyglycerinrest oder
R ist ein Kohlehydratrest, ausgewählt aus den Monosacchariden Glucose, Fructose, Ribose, Desoxyribose, Inosit, aus den Oligosacchariden Saccharose, Raffinose, Gentianose, Malecitose, Stachyose,, Verbascose, aus den Polysacchariden Stärke, Lichenine, Glykogen, Dextrine, Cyclodextrine , Dextrane, Inuline, Fruktosane, Lävane, Mannane, Galaktane, Xylane, Arabane, Pektine, Makropolysaccharide, Glycoproteide, aus Polyuridenylsäure, Polyglucuronsäure, Polygalacturonsäure, Polymannuronsäure und/oder Alginsäure und deren Derivaten;
A fehlt oder
A ist eine Alkyl-, Alkoxy-, Acyl-, Acylamin-, Alkylamingruppe, bei denen die Zahl der Kohlenstoffatome der Alkoxy-, Acyl-, Acylamin-, Alkylgruppe im Bereich von 1 bis 4 liegt;
B ist ein phosphorhaltiger Rest, ausgewählt unter Monophosphat, Diphosphat, Polyphosphat, Phosphonat, Thiophosphat, Thiophosphonat, oder ein carboxylat-, sulfat-, sulfonat-, mercapto-, silantriol-oder trialkoxysilanhaltiger Rest;
(ii) phosphatgruppenhaltigen Nucleotiden Mono-, Di-, Triphosphorsäureester oder Mono-, Di-, Tri-phosphorsäureester- chloriden von Adenosin, Guanosin, Cytidin, Uridin, Thymidin, Desoxyadenosin, Desoxyguanosin, Desoxycytidin, Desoxythymidin, Inosin, Pyrimidin, Cytosin, Uracil, Thymin, Purin, Adenin, Guanin, Methylcytosin, 5-Hydroxymethyl-cytosin, 2-Methyladenin, 1-Methylguanin, Thiamin, Flavin, Riboflavin sowie Pyridoxalphosphat, Pyridoxaminphosphat, Ribonucleinsäure, Ribonucleinsäuresequenzen, Desoxyribonucleinsäuren, Desoxyribonucleinsäuresequenzen und/oder
(iii) Alkyl-, Aryl-, und/oder Alkyl-aryl-polyethylenglykol- phosphaten oder -phosphonaten, wobei die Zahl der Kohlenstoffatome der Alkylgruppe im Bereich von 5 bis 22, und die Zahl der Ethylenglykolgruppen im Polyethylenglykol im Bereich zwischen 4 und 1000 liegt,
(iv) stickstoffhaltigen Polysacchariden, ausgewählt unter Mucopolysacchariden, Glykoproteiden, Chitinen, sowie deren Denaturierungsprodukten.

7. Verfahren zur Herstellung von stabilisierten superparamagnetischer Teilchen unter Herstellung von superparamagnetischen Eindomänenteilchen aus Eisenoxid-, Eisenmischoxid- oder Eisen mit einer Teilchengröße im Bereich zwischen 3 und 20 Nanometer durch Fällung aus wäßrigen Eisensalzlösungen mit Alkalilauge oder Ammoniakwasser, dadurch gekennzeichnet, daß die Dispersion aus superparamagnetischen Eindomänenteilchen mit Salzsäure auf einen pH-Wert zwischen 1 und 7 eingestellt und unter erhöhter Temperatur und gegebenfalls erhöhtem Druck zur Aggregation gebracht wird zu stabilen, abbaubaren superparamagnetischen Aggregaten mit einem Teilchengröße im Bereich zwischen 10 und 1000 Nanometer mit definiertem Verhalten im Magnetfeld, und diese gereinigt werden, wobei die superparamagnetischen Aggregate mit 20 bis 50 Gew.-% organischer Substanz behandelt werden, so daß sie auf ihrer Oberfläche mono- und/oder polyhydroxylgruppenhaltige aromatische Substanzen, silikatgruppenhaltige Substanzen der orthokieselsäure und deren Kondensationsprodukte mit zwei und mehrwertigen anorganischen Ionen und/oder organischen Säuren und Basen, sowie phosphatgruppenhaltige Substanzen der Ortho- oder Metaphosphorsäure und deren Kondensationsprodukte und gegebenenfalls organische Substanzen der Gruppe der phosphat-, diphosphat-, carboxylat-, polyphosphat-, thiophosphat-, phosphonat-, thiophosphonat-, sulfat-, sulfonat-, mercapto-, carboxylat-, silantriol-, trialkoxysilangruppenhaltigen Polyalkylenglykole, Polyglycerine oder Kohlehydrate, der Gruppe der phosphatgruppenhaltigen Nucleotide, deren oligomere oder Polymere, der Gruppe der Alkyl-, Aryl-, und/oder Alkyl-aryl-polyethylenglykol-phosphate oder -phosphonate, der Gruppe der Mucopolysaccharide, Glykoproteide, Chitine, sowie deren Derivate und Denaturierungsprodukte, gebunden Tragens die weitere Bindungsstellen haben können und in an sich bekannter Weise gereinigt und gegebenfalls noch mit pharmakologisch oder diagnostisch Wirksamen substanzen in an sich bekannter Weise gekoppelt werden.

8. Pharmakologisch wirksame Zubereitung, bestehend aus einem pharmakologisch annehmbaren Träger und superparamagnetischen Teilchen nach Anspruch 1 mit einer Teilchengröße im Bereich zwischen 10 und 1000 nm, gegebenenfalls in Verbindung mit einer gewebespezifischen Bindungssubstanz, einer pharmakologisch wirksamen Substanz, einer pharmakologisch wirksamen Zelle, einem pharmakologisch wirksamen Komplexbildner, einer zellfusionvermittelnden Substanz und/oder einer gentransfer-vermittelnden Substanz.

9. Verwendung einer pharmakologisch wirksamen Zubereitung nach Anspruch 8 zur Herstellung eines Mittels zur Tumorschädigung, zur Immunsteigerung, zum magnetischen drug targeting, zur Zellfusion, zum Gentransfer, als Kontrastmittel, sowie als magnetischen Ionenaustauscher und magnetische Adsobentien für Separationsverfahren, gegebenfalls unter Einwirkung von Magnetfeldern.

## Claims

1. Superparamagnetic particles, comprising particles selected from the group consisting of
(a) small superparamagnetic one-domain particles of iron hydroxide, iron oxide hydrate, iron oxide, mixed iron oxide or iron with a particle size ranging between 3 and 50 nanometres; and
(b) dispersion stable, physisological degradable aggregates with a particle size ranging between 10 and 1000 nanometres, whereby the aggregate consist of several small superparamagnetic one-domain particles of iron hydroxide, iron oxide hydrate, iron oxide, mixed iron oxide or iron with a particle size ranging between 3 and 50 nanometres;
and their mixtures;
wherein to the surfaces of the particles or aggregates or mixtures thereof are bound
(c) substances of the group of
(i) mono or polyhydroxylic group-containing or both groups containing aromatic substances, selected from among benzenoids, coumarins, lignans, terphenyls, flavonoids, tannins, xanthenes, benzophenones, naphthalenes, naphthoquinones, anthraquinones, anthracyclines, polycyclic condensated aromatic compounds and their phosphate, diphosphate, polyphosphate, thiophosphate, phosphonate, thiophosphonate, carboxylate, sulphate, mercapto or silantriol group-containing derivatives;
(ii) silicate group-containing substances of ortho-silicic acid and their condensation products with bivalent and polyvalent inorganic ions or with organic acids or bases or mixtures thereof; and
(iii) phosphate group-containing substances of ortho- or metaphosphoric acid and their condensation products, as well as their heterocondensation products and their water-insoluble salt compounds with inorganic ions and basic group-containing organic compounds;
and mixtures of substances of the groups (i)-(iii);
and optionally, in addition to the substances (c) and on their binding sites are chemically bound organic substances (d), of the group of
(iv) phosphate, diphosphate, carboxylate, polyphosphate, thiophosphate, phosphonate, thiophosphonate, sulphate, sulphonate, mercapto, carboxylate, silantriol, trialkoxy silane group-containing polyalkylene glycols and carbohydrates; and
(v) phosphate group-containing nucleotides, their oligomers or their polymers; and
(vi) the alkyl, aryl, alkyl-aryl-polyethylene glycol phosphates or -phosphonates, phosphates or phosphonates of the substance copolymerides from polyethylene glycol (PEG) and polypropylene glycol (PPG), selected from among the substance copolymerides (PEG)ₙ-(PEG)ₘ, (PEG)ₙ-(PPG)ₘ-(PEG)ₙ, (PPG)ₘ-(PEG)ₙ-(PPG)ₘ;
(vii) nitrogenous polysaccharides, selected from among mucopolysaccharides, glycoproteids, chitins, as well as their derivatives and denaturation products;
and mixtures of substances of the groups (iv)-(vii).

2. Superparamagnetic particles according to claim 1, wherein the superparamagnetic one-domain particles (a) and the particles of the stable, degradable aggregates (b) comprise iron hydroxide, iron oxide hydrate,
γ-Fe₂O₃, Fe₃O₄, from the mixed iron oxides of the general formula mMo.nFe₂O₃, where M represents the bivalent metal ions Fe, Co, Ni, Mn, Be, Mg, Ca, Ba, Sr, Cu, Zn, Pt or mixtures thereof, from the mixed oxides of the general formula mFe₂O₃.nMe₂O₃, where Me represents the trivalent metal ions Al, Cr, Bi, rare earth metals or mixtures of them, or of iron.

3. Superparamagnetic particles according to one of claims 1 and 2, wherein the substances c) are selected from among mono or polyhydroxylic group-containing or both groups containing aromatic benzenoids, coumarins, lignans, terphenyls, flavonoids, tannins, xanthenes, benzophenones, naphthalenes, naphthoquinones, anthraquinones, anthracyclines, polycyclic condensated aromatic compounds and their phosphate, diphosphate, polyphosphate, thiophosphate, phosphonate, thiophosphonate, carboxylate, sulphate, mercapto or silantriol group-containing derivatives.

4. Superparamagnetic particles according to one of claims 1 to 3, wherein the substances c) are selected from among the silicate group-containing substances of ortho-silicic acid and their condensation products with bivalent and polyvalent inorganic ions, selected from among Al, Au, Bi, Cr, J, Mo, P, Pt, Se, Tc, Ti, Y, Zr, rare earth metals and/or organic acids and bases.

5. Superparamagnetic particles according to one of claims 1 to 3, wherein the substances (c) are selected from among the phosphate group-containing substances of ortho or metaphosphoric acid and their condensation products, as well as their heterocondensation products and their water-insoluble sodium compounds with inorganic ions, organic bases and basic group-containing organic compounds.

6. Superparamagnetic particles according to claim 1, wherein in addition to the substances (c), to their surfaces are bound organic substances (d), selected from among (i) the compounds of the general formula
X - R - A - B
where X represents a functional group selected from among the group of alkoxy, monoalkylamino, dialkylamino, trialkylamino and alkylthio group, in which the number of carbon atoms in the alkyl part of this group ranges from 1 to 4, or represents a functional group, selected from among the hydroxyl, amine, aldehyde, dimethylacetal, diethylacetal, epoxy, thiol, carboxy, 4,6-dichlortriazine, hydroxamic acid, isocyanate, acyl acid, anhydride, diazonium salt, iminocarbonate and toluol sulphonate group;
R is a polyethylene glycol residue (PEG)ₙ, a water-miscible polypropylene glycol residue (PPG)ₘ or a water-miscible substance copolymeride residue of polyethylene glycol (PEG) and polypropylene glycol (PPG), selected from among the substance copolymerides (PEG)ₙ-(PEG)ₘ, (PEG)ₙ-(PPG)ₘ-(PEG)ₙ, (PPG)ₘ-(PEG)ₙ-(PPG)ₘ whereby n and m are positive whole numbers, selected for PEG in the range of 4 to 1000, for PPG in the range of 3 to 12 and for PEG-PPG substance copolymerides in the range of 3 to 140,
R is a polyglycerin residue or
R is a carbohydrate residue, selected from among the monosaccharides glucose, fructose, ribose, desoxyribose, inosite, from the oligosaccharides saccharose, raffinose, gentianose, malecitose, stachyose, verbascose, from the polysaccharides starch, lichenins, glycogen, dextrins, cyclodextrins, dextrans, inulins, fructosans, levans, mannans, galactans, xylans, arabans, pectins, macropolysaccharides, glycocoproteids, from polyuridenylic acid, polyglucuronic acid, polygalacturonic acid, polymannuronic acid, alginic acid; and derivatives thereof;
A is missing or
A is an alkyl, alkoxy, acyl, acylamine, alkylamine-group in which the number of carbon atoms of the alkoxy, acyl, acylamine, alkyl group is in the range of 1 to 4;
B is a residue containing phosphorus, selected from among monophosphate, diphosphate, polyphosphate, phosphonate, thiophosphate, thiophosphonate, or a residue containing carboxylate, sulphate, sulphonate, mercapto, silantriol or trialkoxysilan;
(ii) phosphate group containing nucleotides mono, di, tri phosphoric ester or mono, di, tri phosphoric ester chlorides of adenosine, guanosine, cytidine, uridine, thymidine, desoxyadenosine, desoxyguanosine, desoxycytidine, desoxythymidine, inosine, pyrimidine, cytosine, uracil, thymine, purine, adenine, guanine, methylcytosine, 5-hydroxymethylcytosine, 2-methyladenine, 1-methylguanine, thiamine, flavin, riboflavin as well as pyridoxal phosphate, pyridoxamine phosphate, ribonucleic acid, ribonucleic acid sequences, desoxyribonucleic acids, desoxyribonucleic acid sequences; (iii) alkyl, aryl and/or alkyl-aryl-polyethylene glycol phosphates or -phosphonates, in which the number of carbon atoms of the alkyl group ranges from 5 to 22, and the number of ethylene glycol groups in the polyethylene glycol ranges from 4 to 1000;
(iv) nitrogenous polysaccharides, selected from among mucopolysaccharides, glycoproteids, chitins, as well as their denaturation products;
and mixtures of substances of the groups (i)-(v).

7. Process for manufacturing stabilised superparamagnetic particles by manufacturing superparamagnetic one-domain particles of iron oxide, mixed iron oxide or iron with a particle size ranging from 3 to 20 nanometres by precipitation from aqueous ferric salt solutions with alkaline lye or aqueous ammonia, which comprise that the dispersion of superparamagnetic one-domain particles is set to a pH value between 1 and 7 with hydrochloric acid and with increased temperature, and optionally with increased pressure, is brought to aggregation to stable, degradable superparamagnetic aggregates with a particle size ranging from 10 to 1000 nanometres with defined behaviour in the magnetic field, and these are cleaned, whereby the superparamagnetic aggregates are treated with an organic substance 20 to 50 % by weight, so that to their surfaces are bound mono or polyhydroxylic group-containing or both groups containing aromatic substances, silicate group-containing substances of ortho-silicic acid and their condensation products with bivalent and polyvalent inorganic ions or organic acids and bases or both, as well as phosphate group-containing substances of ortho or metaphosphoric acid and their condensation products and optionally organic substances of the group of phosphate, diphosphate, carboxylate, polyphosphate, thiophosphate, phosphonate, thiophosphonate, sulphate, sulphonate, mercapto, carboxylate, silantriol, trialkoxy silan group-containing polyalkylene glycols, polyglycerins or carbohydrates, of the group of phosphate group-containing nucleotides, their oligomers or polymers, the group of the alkyl, aryl, and/or alkyl-aryl-polyethylene glycol phosphates or -phosphonates, the group of the mucopolysaccharides, glycoproteids, chitins, as well as their derivatives and denaturation products - these can have further binding sites and are cleaned in the known manner and optionally can additionally be coupled with pharmacologically or diagnostically active substances in the known manner.

8. Pharmacologically active preparation, consisting of a pharmacologically acceptable carrier and superparamagnetic particles according to claim 1 with the particle size ranging from 10 to 1000 nm, optionally coupled with a tissue-specific binding substance, a pharmacologically active substance, a pharmacologically active cell, a pharmacologically active complexing agent, a cell-fusioning substance or a gene-transfer medium or a mixture thereof.

9. Use of a pharmacologically active preparation according to claim 8 for manufacturing of an agent to destroy tumours, to stimulate the immune system, for magnetic drug targeting, for cell fusion, for gene transfer, as a contrast medium, as well as a magnetic ion exchanger and as magnetic adsorbents for separation processes, optionally with the effect of magnetic fields.

## Revendications

1. Particules superparamagnétiques, caractérisées en ce qu'elles comprennent des particules sélectionnées dans le groupe comprenant
a) de petites particules superparamagnétiques à un domaine constituées d'hydroxyde de fer, d'hydrate d'oxyde de fer, d'oxyde de fer, d'oxyde de fer mixte ou de fer, d'une granulométrie comprise entre 3 et 50 nanomètres,
b) des agrégats stables dégradables d'une granulométrie comprise dans l'intervalle de 10 à 1000 nanomètres, les agrégats étant constitués de plusieurs petites particules superparamagnétiques à un domaine à base d'hydroxyde de fer, d'hydrate d'oxyde de fer, d'oxyde de fer, d'oxyde de fer mixte ou de fer, d'une granulométrie comprise dans l'intervalle de 3 à 50 nanomètres, et de leurs mélanges,
c) les particules portant sur leur surface des substances sélectionnées dans le groupe comprenant
i) des substances aromatiques renfermant des groupements mono- et/ou polyhydroxyle, sélectionnés parmi les benzénoïdes, les coumarines, les lignanes, les terphényles, les flavonoïdes, les tanins, les xanthones, les benzophénones, les naphtalènes, les naphtoquinones, les anthraquinones, les anthracyclines, les composés aromatiques condensés polycycliques et leurs dérivés contenant des groupements phosphate, diphosphate, polyphosphate, thiophosphate, phosphonate, thiophosphonate, carboxylate, sulfate, mercapto ou silanetriol;
ii) des substances contenant des groupements silicate issues de l'acide orthosilicique et de ses produits de condensation avec des ions organiques bi- et plurivalents ou avec des acides ou des bases organiques ou des mélanges de ceux-ci;
iii) des substances contenant des groupements phosphate issues de l'acide ortho- ou métaphosphorique et de ses produits de condensation ainsi que de ses produits d'hétérocondensation et de composés de sels insolubles dans l'eau avec des ions inorganiques et des composés contenant des groupes basiques;
et leurs mélanges,
les particules portant éventuellement, liées chimiquement sur leur surface, en plus des substances (c), des substances organiques (d) sélectionnées dans le groupe comprenant:
iv) des polyalkylène glycols et des hydrates de carbone contenant des groupements phosphate, diphosphate, carboxylate, polyphosphate, thiophosphate, phosphonate, thiophosphonate, sulfate, sulfonate, mercapto, carboxylate, silanetriol et trialcoxysilane;
v) des nucléotides contenant des groupements phosphate, leurs oligomères ou leurs polymères;
vi) des phosphates ou phosphonates de polyalkylèneglycols alkylés, arylés et/ou alkylarylés, des phosphates ou phosphonates des bloc-copolymères de polyéthylèneglycol (PEG) et de polypropylène glycol (PPG) choisis parmi les bloc-copolymères (PEG)ₙ-(PPG)ₘ, (PEG)ₙ- (PPG)ₘ- (PEG)ₙ, (PPG)ₘ-(PEG)ₙ-(PPG)ₘ;
vii) des polysaccharides contenant de l'azote, sélectionnés parmi les mucopolysaccharides, les glycoprotéides, les chitines, ainsi que leurs dérivés et produits de dénaturation;
et leurs mélanges.

2. Particules superparamagnétiques selon la revendication 1, caractérisées en ce que les particules superparamagnétiques à un domaine (a) et les particules des agrégats stables dégradables (b) se composent d'hydroxyde de fer, d'hydrate d'oxyde de fer, de γ-Fe₂O₃, de Fe₃O₄, des oxydes de fer mixtes de la formule générale mMO.nFe₂O₃, dans laquelle M représente les ions métalliques bivalents Fe, Co, Ni, Mn, Be, Mg, Ca, Ba, Sr, Cu, Zn, Pt ou des mélanges de ceux-ci; des oxydes mixtes de la formule générale mFe₂O₃.nMe₂O₃, dans laquelle Me représente les ions métalliques trivalents Al, Cr, Bi, des métaux terreux rares ou des mélanges de ceux-ci; ou de fer.

3. Particules superparamagnétiques selon l'une des revendications 1 à 2, caractérisées en ce que les substances (c) sont sélectionnées parmi les benzénoïdes, les coumarines, les lignanes, les terphényles, les flavonoïdes, les tanins, les xanthones, les benzophénones, les naphtalènes, les naphtoquinones, les anthraquinones, les anthracyclines, les composés aromatiques condensés polycycliques et leurs dérivés contenant des groupements phosphate, diphosphate, polyphosphate, thiophosphate, phosphonate, thiophosphonate, carboxylate, sulfate, mercapto ou silanetriol, contenant des groupements mono- et/ou polyhydroxyle.

4. Particules superparamagnétiques selon l'une des revendications 1 à 3, caractérisées en ce que les substances (c) sont sélectionnées parmi les substances contenant des groupements silicate issues de l'acide orthosilicique et de ses produits de condensation avec des ions inorganiques bi- ou plurivalents choisis parmi Al, Au, Bi, Cr, J, Mo, P, Pt, Se, Tc, Ti, Y, Zr, des métaux terreux rares et/ou des acides et des bases organiques.

5. Particules superparamagnétiques selon l'une des revendications 1 à 4, caractérisées en ce que les substances (c) sont sélectionnées parmi les substances contenant des groupes phosphate issues de l'acide ortho- ou méthaphosphorique et de leurs produits de condensation ainsi que de leurs produits d'hétérocondensation et des composés de sels insolubles dans l'eau avec des ions inorganiques, des bases organiques et des composés organiques contenant des groupes basiques.

6. Particules superparamagnétiques selon la revendication 1, caractérisées en ce qu'en plus des substances (c), d'autres substances organiques (d) sont encore attachées à leur surface, sélectionnées (i) parmi les composés de la formule générale:
X - R - A - B
dans laquelle X représente un groupement fonctionnel sélectionné parmi les groupements alcoxy, monoalkylamino, dialkylamino, trialkylamino et alkylthio, dans lesquels le nombre des atomes de carbone dans la partie alkyl de ces groupements est de l'ordre de 1 et de 4, ou représente un groupement fonctionnel choisi parmi les groupements hydroxyle, amine, aldéhyde, diméthylacétal, diéthylacétal, époxy, thiol, carboxy, dichloro-4,6 triazine, acide hydroxamique, isocyanate, acylazide, anhydride, sel de diazonium, iminocarbonate et toluolsulfonate; R est un radical de polyéthylèneglycol (PEG)ₙ, un radical de polypropylène glycol miscible avec l'eau (PPG)ₘ, ou un radical de bloc-copolymère constitué de polyéthylèneglycol (PEG) et de polypropylène glycol (PPG), sélectionné parmi les bloc-copolymères (PEG)ₙ-(PPG)ₘ, (PEG)ₙ-(PPG)ₘ-(PEG)ₙ-(PPG)ₘ, n et m sont des nombres entiers positifs sélectionnés pour PEG dans l'intervalle de 4 à 1000, pour PPG dans l'intervalle de 3 à 12, et pour les bloc-copolymères PEG-PPG- dans l'intervalle de 3 à 140,
R est un radical de polyglycérol ou
R est un radical d'hydrate de carbone, sélectionné parmi les monosaccharides glucose, fructose, ribose, désoxyribose, inosite, parmi les oligosaccharides saccharose, raffinose, gentianose, malécitose, stachyose, verbascose, parmi les polysaccharides amidon, lichénine, glycogène, dextrine, cyclodextrine, dextrane, inuline, fructosane, lévane, mannane, galactane, xylane, arabane, pectine, macropolysaccharides, glycoprotéides, parmi l'acide polyuridénylique, l'acide polyglucuronique, l'acide polygalactucuronique, l'acide polymannuronique et/ou l'acide alginique et leurs dérivés;
A est manquant ou
A est un groupement alkyle, alcoxy, acyle, acylamine, alkylamine, dans lequel le nombre des atomes de carbone des groupements alcoxy, acyle, acylamine, alkyle est de l'ordre de 1 à 4;
B est un radical contenant du phosphore, sélectionné parmi le monophosphate, le diphosphate, le polyphosphate, le phosphonate, le thiophosphate, le thiophosphonate, ou un radical renfermant un carboxylate, un sulfate, un sulfonate, un mercapto, un silanetriol ou un trialcoxysilane;
ii) les nucléotides contenant des groupements phosphate, ester d'acide mono-, di-, triphosphorique ou chlorures d'esters d'acide mono-, di, triphosphorique d'adénosine, de guanosine, de cytidine, d'uridine, de thymidine, de désoxyadénosine, de désoxyguanosine, de désoxycytidine, de désoxythymidine, d'inosine, de pyrimidine, de cytosine, d'uracile, de thymine, de purine, d'adénine, de guanine, de méthyl cytosine, d'hydroxyméthyl-5 cytosine, de méthyl-2 adénine, de méthyl-1 guanine, de thiamine, de flavine, de riboflavine, ainsi que le pyridoxal phosphate, le pyridoxamine phosphate, l'acide ribonucléique, des séquences d'acide ribonucléique, des acides désoxyribonucléiques, des séquences d'acide désoxyribonucléique et/ou
iii) des phosphates ou phosphonates de polyéthylèneglycol alkylé, arylé et/ou alkylarylé, le nombre des atomes de carbone du groupement alkyle étant de l'ordre de 5 à 22, et le nombre des groupements éthylèneglycol dans le polyéthylèneglycol étant compris entre 4 et 1000,
iv) des polysaccharides contenant de l'azote, sélectionnés parmi les mucopolysaccharides, les glycoprotéides, les chitines, ainsi que leurs produits de dénaturation.

7. Procédé de préparation de particules superparamagnétiques via la préparation de particules superparamagnétiques à un domaine à base d'oxyde de fer, d'oxyde de fer mixte ou de fer, d'une granulométrie comprise entre 3 et 20 nanomètres, par précipitation au départ de solutions de sels de fer aqueuses avec de la lessive de soude caustique ou de l'eau ammoniacale, caractérisé en ce que la dispersion de particules superparamagnétiques à un domaine est ajustée sur un pH compris entre 1 et 7, avec de l'acide chlorhydrique, et est amenée à s'agréger à température accrue et le cas échéant à pression accrue pour former des agrégats superparamagnétiques stables dégradables d'une granulométrie comprise entre 10 et 1000 nanomètres, présentant dans le champ magnétique un comportement défini, et en ce que ceux-ci sont purifiés, les agrégats superparamagnétiques étant traités avec 20 à 50 % en poids de substance organique, de telle sorte qu'ils portent, liés à leur surface, des subtances aromatiques contenant des groupements mono- ou polydroxyle, des substances contenant des groupements silicate issues de l'acide orthosilicique et de ses produits de condensation avec des ions inorganiques bi- et plurivalents et/ou des acides et des bases organiques, ainsi que des substances contenant des groupements phosphate issues de l'acide ortho- ou métaphosphorique et de leurs produits de condensation et le cas échéant des substances organiques du groupe comprenant les polyalkylène glycols, polyglycérols ou hydrates de carbone contenant des groupements phosphate, diphosphate, carboxylate, polyphosphate, thiophosphate, phosphonate, thiophosphonate, sulfate, sulfonate, mercapto, carboxylate, silanetriol, trialcoxysilane, du groupe des nucléotides contenant des groupements phosphate, leurs oligomères ou polymères, du groupe des phosphates ou phosphonates de polyéthylèneglycol alkylé, arylé et/ou alkylarylé, du groupe des mucopolysaccharides, glycoprotéides, chitines ainsi que leurs dérivés et produits de dénaturation, qui peuvent avoir d'autres sites d'attachement et qui peuvent être purifiés de la manière connue en soi et éventuellement encore couplés, de la manière connue en soi, avec des substances actives sur le plan pharmacologique ou en diagnostic.

8. Préparation à activité pharmacologique, comprenant un transporteur acceptable du point de vue pharmacologique et des particules superparamagnétiques selon la revendication 1, d'une granulométrie comprise dans l'intervalle de 10 et 1000 nm, éventuellement en combinaison avec une substance de liaison spécifique du tissu, une substance à activité pharmacologique, une cellule à activité pharmacologique, un complexant à activité pharmacologique, une substance favorisant la fusion des cellules et/ou une substance favorisant le transfert de gènes.

9. Utilisation d'une préparation à activité pharmacologique conforme à la revendication 8, en vue de la préparation d'un agent pour la lésion de tumeurs, pour le renforcement des défenses immunitaires, pour le pharmaco-ciblage magnétique, pour la fusion des cellules, pour le transfert de gènes, comme agent de contraste, ainsi que comme échangeurs d'ions et agents d'adsorption pour procédés de siparation, le cas échéant sous l'influence de champs magnétiques.
